# EUROPEAN PATENT APPLICATION

(11) **EP 4 265 639 A2**
(43) Date of publication of application: **25.10.2023**
(21) Application number: 21906991.1
(22) Date of filing: 10.12.2021
(51) Int. Cl.: C07K 16/28, A61P 35/00, A61K 39/00

(54) **ANTI-HVEM ANTIBODY, AND COMPOSITION AND METHOD ASSOCIATED WITH SAME**

(30) Priority: 15.12.2020 KR 20200175769; 19.03.2021 KR 20210035734
(71) Applicant: Medytox Inc., Chungcheongbuk-do 28126 (KR)
(72) Inventor: LEE, Deokjae, Yongin-si Gyeonggi-do 16978 (KR); SONG, Suk-yoon, Siheung-si Gyeonggi-do 14984 (KR)
(74) Representative: Brevalex
(86) International application number: PCT/KR2021/018707
(87) International publication number: WO 2022/131687

(57) **Abstract**

Provided are an anti-HVEM monoclonal antibody that binds to human HVEM with high affinity, and inhibits the binding of HVEM to BTLA but promotes the binding of HVEM to LIGHT, and thus can be used alone or in combination with other therapeutic agents to treat solid tumor or hematologic malignancy, and a composition and a method which are related to the antibody.

## Description

### Technical Field

The present disclosure relates to an anti-HVEM antibody, and particularly to an anti-HVEM antibody that selectively inhibits or promotes the binding of HVEM to a specific ligand, and a composition and a method which are related to the antibody.

### Background Art

Recently, a novel co-inhibitory pair, Herpes Virus Entry Mediator (HVEM) and B and T lymphocyte attenuator (BTLA), has been highlighted in anti-tumor immune responses. These two molecules can be expressed by many immune cells, including T cells, in which signaling through BTLA is associated with inhibition of the activation of these immune cells. In addition, the HVEM network includes other additional partners, such as LIGHT (tumor necrosis factor superfamily member 14 (TNFSF14)), lymphotoxin α (LT α), or CD160. Like BTLA, binding of HVEM to CD160 on T cells is associated with inhibition of the activation thereof. Meanwhile, stimulation of HVEM on T cells by a ligand is associated with cell proliferation, survival, and production of inflammatory cytokines, such as IL-2 and IFN-γ. Several clinical studies have shown that HVEM expression is upregulated in many types of cancers, including colorectal cancer, esophageal cancer, gastric cancer, liver cancer, breast cancer, or lymphoma. In these studies, high levels of HVEM expression by tumors were associated with a worse prognosis and lower survival. Moreover, HVEM expression by tumors was also associated with a reduction in the number of CD4 and CD8 tumor-infiltrating leukocytes (TILs).

International Publication NOs. WO2008/145754 and WO2008/146101 disclose a monoclonal antibody that that does not block the binding of HVEM to BTLA, LIGHT, and gD or blocks the binding of HVEM to LIGHT, as an antibody against HVEM which can be used to treat hematologic malignancies and autoimmune diseases.

International Publication NOs. WO2014/183885 and WO2014/184360 disclose an antibody or fragment thereof that increase the proliferation of Vγ9Vδ2 T cells, as an antagonist of the BTLA/HVEM interaction. The antibody is HVEM 18.10 produced by a hybridoma deposited with Accession No. CNCM 1-4752 in the Collection Nationale de Cultures de Microorganismes as a monoclonal antibody against HVEM. HVEM 18.10 is a mouse IgG1 anti-human HVEM mAb (murine anti-human HVEM mAb) produced in ascites and purified by protein A binding and elution. The antibody has the property of inhibiting all binding of HVEM to BTLA, LIGHT, and gD. This patent discloses that the antibody can be used for the treatment of hematological malignancies, such as lymphoma or solid tumor.

International Publication No. WO2020/222235 discloses an antibody that specifically binds to HVEM that inhibits the interaction between HVEM and BTLA and activates downstream signaling through HVEM in cells.

To date, an anti-HVEM antibody that inhibits the binding of HVEM to BTLA or gD but promotes the binding of HVEM to LIGHT has not been known.

### Disclosure

### Technical Solution

An object of the present disclosure is to provide an anti-HVEM antibody that binds to human HVEM with high affinity, and inhibits the binding of HVEM to BTLA and/or gD but promotes the binding of HVEM to LIGHT.

Another object of the present disclosure is to provide an isolated nucleic acid encoding the antibody.

Another object of the present disclosure is to provide a host cell including the nucleic acid.

Another object of the present disclosure is to provide a method of producing the antibody by culturing the host cell.

Another object of the present disclosure is to provide a pharmaceutical composition including the antibody as an active ingredient.

Another object of the present disclosure is to provide a method of treating a disease by administering the antibody to a subject.

### Technical Solution

One aspect of the present disclosure relates to an anti-HVEM monoclonal antibody that binds to human HVEM with a dissociation constant (Kd) of 500 pM or less, and inhibits the binding of HVEM to BTLA but promotes the binding of HVEM to LIGHT.

Another aspect of the present disclosure relates to an isolated nucleic acid encoding the antibody.

Another aspect of the present disclosure relates to a host cell including the nucleic acid.

Another aspect of the present disclosure relates to a method of producing an antibody, including culturing the host cell under conditions suitable for the expression of a nucleic acid encoding an anti-HVEM antibody.

Another aspect of the present disclosure relates to a pharmaceutical composition for treating cancer, including the antibody as an active ingredient and a pharmaceutically acceptable carrier.

Another aspect of the present disclosure relates to a method of treating cancer in a subject, including administering a therapeutically effective amount of the antibody to a subject in need of cancer treatment.

### Advantageous Effects

According to the present disclosure, provided are: an anti-HVEM monoclonal antibody that binds to human HVEM with high affinity, and inhibits the binding of HVEM to BTLA but promotes the binding of HVEM to LIGHT, and thus can be used alone or in combination with other therapeutic agents to treat solid tumor or hematologic malignancy; and a composition and a method which are related to the anti-HVEM monoclonal antibody.

### Description of Drawings

FIGS. 1A and 1B illustrate the binding affinity of anti-HVEM antibodies to human HVEM and mouse HVEM.
FIGS. 2A, 2B, 2C, and 2D are graphs showing cross-reactivity of anti-HVEM antibodies with human, cynomolgus monkey, and mouse HVEM.
FIGS. 3A, 3B, and 3C illustrate the results of measuring the binding affinity of anti-HVEM antibodies for human HVEM in octets.
FIG. 4 illustrates the inhibition rate (%) of the HVEM-BTLA interaction of anti-HVEM antibodies.
FIG. 5 illustrates the inhibition rate (%) of the HVEM-BTLA interaction of anti-HVEM antibodies.
FIG. 6 illustrates the inhibition rate (%) of the HVEM-gD interaction of anti-HVEM antibodies.
FIG. 7 illustrates the HVEM-BTLA immune checkpoint inhibitory activity of anti-HVEM antibodies.
FIG. 8 is a graph showing the tumor growth inhibitory effect of an anti-HVEM antibody in an animal model.
FIG. 9 illustrates the tumor growth inhibitory effect of the combined use of an anti-HVEM antibody and an anti-CTLA4 antibody in an animal model.
FIG. 10 illustrates the tumor growth inhibitory effect of the combined use of an anti-HVEM antibody and an anti-PD1 antibody in an animal model.
FIG. 11 illustrates the tumor growth inhibitory effect of anti-HVEM antibodies in a humanized animal model.
FIG. 12 illustrates the tumor growth inhibitory effect of the combined use of an anti-HVEM antibody and an anti-PD-L1 antibody in a humanized animal model.

### Best Mode

### Definition

The term "antibody" includes whole antibodies and any antigen-binding fragment or single chains thereof. The "antibody" includes a glycoprotein including at least two heavy (H) chains and two light (L) chains inter-connected by disulfide bonds or an antigen-binding portion thereof. Each heavy chain consists of a heavy chain variable region (VH or HV) and a heavy chain constant region. The heavy chain constant region consists of four regions: CH1, hinge, CH2, and CH3. Each light chain consists of a light chain variable region (VL or LV) and a light chain constant region. The light chain constant region consists of one domain, CL. The VH and VL regions can be subdivided into hypervariable regions (HVRs), termed complementarity determining regions (CDRs), interspersed with regions that are more conserved, termed framework regions (FR). Each VH and VL consists of three CDRs and four FRs arranged from the amino-terminus to the carboxy-terminus in the following order: FR1, CDR1, FR2, CDR2, FR3, CDR3, FR4. The variable regions of the heavy and light chains contain a binding domain that interacts with an antigen. The constant regions may mediate the binding of immunoglobulin to host tissues or factors, including various cells of the immune system (e.g., effector cells) and the first component (Clq) of the classical complement system.

The term "monoclonal antibody" refers to an antibody derived from a population of substantially homogeneous antibodies, wherein the individual antibodies included in the population are identical and/or bind to the same epitope, except for variant antibodies present in trace amounts. For example, the monoclonal antibody may be prepared by a variety of techniques, including a hybridoma method, a recombinant DNA method, a phage display method, and a method using a transgenic animal that contains all or part of the human immunoglobulin locus.

The term "binding affinity" refers to the inherent binding affinity reflecting the 1:1 interaction between members of a binding pair (e.g., antibody and antigen). The affinity of molecule X for its partner Y can usually be expressed as a dissociation constant (Kd). Affinity can be measured by common methods known in the art, including those described herein.

The terms "anti-HVEM antibody" and "antibody that binds to HVEM" refer to an antibody capable of binding to HVEM with sufficient affinity so that the antibody is effective as a diagnostic agent and/or a therapeutic agent in targeting HVEM.

An "antibody that binds to the same epitope" as a reference antibody refers to an antibody that blocks binding of the reference antibody to an antigen by 50% or more in a competition assay, and the reference antibody blocks binding of the antibody to an antigen by 50% or more in a competition assay.

The term "human antibody" refers to an antibody that possesses an amino acid sequence corresponding to that of an antibody produced by a human or a human cell or derived from a non-human source that uses human antibody repertoires or other human antibody-encoding sequences.

The term "humanized antibody" refers to a molecule having an antigen-binding site that is substantially derived from an immunoglobulin of a non-human species, wherein the remaining immunoglobulin structure of the molecule is based upon the structure and/or sequence of a human immunoglobulin.

The term "chimeric antibody" refers to an antibody in which a portion of the heavy and/or light chain is derived from a particular source or species, while the remainder of the heavy and/or light chain is derived from a different source or species.

The term "isolated nucleic acid encoding an anti-HVEM antibody" refers to at least one nucleic acid molecule encoding the heavy and light chains (or fragments thereof) of the antibody.

The term "host cell" refers to a cell into which an exogenous nucleic acid has been introduced. Host cells include transformants and transformed cells, which include the primary transformed cell and progeny derived therefrom regardless of the number of passages.

The term "pharmaceutical composition" refers to a preparation containing an active ingredient. The term "preparation" means that, in addition to the active ingredient, at least one additional ingredient is present in the pharmaceutical composition. The pharmaceutical composition is a preparation suitable for administration to a subject, such as a human patient. The pharmaceutical composition may be in lyophilized form, for example a solution formed after reconstitution of the lyophilized pharmaceutical composition with saline or water, or in the form of a solution that does not require reconstitution. The pharmaceutical composition may be liquid or solid.

The term "administration" or "administering" refers to a step of providing a pharmaceutical composition or an active ingredient to a subject. The pharmaceutical composition may be administered via various suitable routes.

The term "therapeutically effective amount" refers to the level, amount or concentration of a pharmaceutical composition including an agent required to treat a disease, disorder or condition without causing significant negative or adverse side effects.

The terms "treat," "treating," or "treatment" refer to, for example, healing injured or damaged tissue, achieving desired therapeutic results by altering, changing, strengthening, ameliorating, improving, and/or beautifying an existing or recognized disease, disorder, or condition; or alleviating, reducing (including partial reduction, substantial reduction, near complete reduction, and complete reduction), resolving or preventing (whether temporarily or permanently) of a disease, a disorder, or a condition. "Prevention" means delaying the onset of a disease, disorder or condition. Prevention may be considered complete when the onset of a disease, disorder, or condition is delayed for a predetermined period of time.

The term "use in combination" refers to any form of administration of two or more different therapeutic agents such that a second therapeutic agent is administered while a previously administered therapeutic agent is still effective in the body. For example, two therapeutic agents are simultaneously effective in a subject, and there may be a synergistic effect of the two therapeutic agents. The different therapeutic agents may be administered concurrently or sequentially in a single formulation or in separate formulations.

### Anti-HVEM Antibody, Nucleic Acid, Host Cell, Production Method

One aspect of the present disclosure relates to an anti-HVEM monoclonal antibody that binds to human HVEM with a dissociation constant (Kd) of 500 pM or less, and inhibits the binding of HVEM to BTLA but promotes the binding of HVEM to LIGHT.

In an embodiment, the antibody may bind to human HVEM with a dissociation constant (Kd) of 400 pM or less, 300 pM or less, 250 pM or less, 200 pM or less, 150 pM or less, 100 pM or less, 50 pM or less, or 30 pM or less.

In an embodiment, the antibody may inhibit the binding of HVEM to BTLA by, for example, 20% or more, for example, 30% or more, for example, 40% or more, for example, 50% or more, for example, 60% or more, for example, 70% or more, for example, 80% or more.

In an embodiment, the antibody may promote the binding of HVEM to LIGHT by, for example, 10% or more, for example, 20% or more, for example, 30% or more, for example, 40% or more, for example, 50% or more.

In an embodiment, the antibody may inhibit the binding of HVEM to gD. The antibody may inhibit the binding of HVEM to gD by, for example, 10% or more, for example, 20% or more, for example, 30% or more.

In an embodiment, the antibody may bind to the same epitope as any one of the following antibodies:
1) an antibody including HCDR1 of SEQ ID NO: 1, HCDR2 of SEQ ID NO: 2, HCDR3 of SEQ ID NO: 3, LCDR1 of SEQ ID NO: 4, LCDR2 of SEQ ID NO: 5, and LCDR3 of SEQ ID NO: 6 (e.g., HVEM011);
2) an antibody including HCDR1 of SEQ ID NO: 9, HCDR2 of SEQ ID NO: 10, HCDR3 of SEQ ID NO: 11, LCDR1 of SEQ ID NO: 12, LCDR2 of SEQ ID NO: 13, and LCDR3 of SEQ ID NO: 14 (e.g., HVEM021);
3) an antibody including HCDR1 of SEQ ID NO: 17, HCDR2 of SEQ ID NO: 18, HCDR3 of SEQ ID NO: 19, LCDR1 of SEQ ID NO: 20, LCDR2 of SEQ ID NO: 21, and LCDR3 of SEQ ID NO: 22 (e.g., HVEM001);
4) an antibody including HCDR1 of SEQ ID NO: 25, HCDR2 of SEQ ID NO: 26, HCDR3 of SEQ ID NO: 27, LCDR1 of SEQ ID NO: 28, LCDR2 of SEQ ID NO: 29, and LCDR3 of SEQ ID NO: 30 (e.g., HVEM004);
5) an antibody including HCDR1 of SEQ ID NO: 33, HCDR2 of SEQ ID NO: 34, HCDR3 of SEQ ID NO: 35, LCDR1 of SEQ ID NO: 36, LCDR2 of SEQ ID NO: 37, and LCDR3 of SEQ ID NO: 38 (e.g., HVEM0017);
6) an antibody including HCDR1 of SEQ ID NO: 41, HCDR2 of SEQ ID NO: 42, HCDR3 of SEQ ID NO: 43, LCDR1 of SEQ ID NO: 44, LCDR2 of SEQ ID NO: 45, and LCDR3 of SEQ ID NO: 46 (e.g., HVEM018);
7) an antibody including HCDR1 of SEQ ID NO: 49, HCDR2 of SEQ ID NO: 50, HCDR3 of SEQ ID NO: 51, LCDR1 of SEQ ID NO: 52, LCDR2 of SEQ ID NO: 53, and LCDR3 of SEQ ID NO: 54 (e.g., HVEM030);
8) an antibody including HCDR1 of SEQ ID NO: 57, HCDR2 of SEQ ID NO: 58, HCDR3 of SEQ ID NO: 59, LCDR1 of SEQ ID NO: 60, LCDR2 of SEQ ID NO: 61, and LCDR3 of SEQ ID NO: 62 (e.g., HVEM032);
9) an antibody including HCDR1 of SEQ ID NO: 65, HCDR2 of SEQ ID NO: 66, HCDR3 of SEQ ID NO: 67, LCDR1 of SEQ ID NO: 68, LCDR2 of SEQ ID NO: 69, and LCDR3 of SEQ ID NO: 70 (e.g., HVEM039);
10) an antibody including HCDR1 of SEQ ID NO: 73, HCDR2 of SEQ ID NO: 74, HCDR3 of SEQ ID NO: 75, LCDR1 of SEQ ID NO: 76, LCDR2 of SEQ ID NO: 77, and LCDR3 of SEQ ID NO: 78 (e.g., HVEM040); and
11) an antibody including HCDR1 of SEQ ID NO: 81, HCDR2 of SEQ ID NO: 82, HCDR3 of SEQ ID NO: 83, LCDR1 of SEQ ID NO: 84, LCDR2 of SEQ ID NO: 85, and LCDR3 of SEQ ID NO: 86 (e.g., HVEM041).

In an embodiment, the antibody may be an antibody that binds to the same epitope as any one of the following antibodies:
1) an antibody including a heavy chain variable region of SEQ ID NO: 7 and a light chain variable region of SEQ ID NO: 8 (e.g., HVEM011);
2) an antibody including a heavy chain variable region of SEQ ID NO: 15 and a light chain variable region of SEQ ID NO: 16 (e.g., HVEM021);
3) an antibody including a heavy chain variable region of SEQ ID NO: 23 and a light chain variable region of SEQ ID NO: 24 (e.g., HVEM001);
4) an antibody including a heavy chain variable region of SEQ ID NO: 31 and a light chain variable region of SEQ ID NO: 32 (e.g., HVEM004);
5) an antibody comprising a heavy chain variable region of SEQ ID NO: 39 and a light chain variable region of SEQ ID NO: 40 (e.g., HVEM017);
6) an antibody including a heavy chain variable region of SEQ ID NO: 47 and a light chain variable region of SEQ ID NO: 48 (e.g., HVEM018);
7) an antibody including a heavy chain variable region of SEQ ID NO: 55 and a light chain variable region of SEQ ID NO: 56 (e.g., HVEM030);
8) an antibody including a heavy chain variable region of SEQ ID NO: 63 and a light chain variable region of SEQ ID NO: 64 (e.g., HVEM032);
9) an antibody including a heavy chain variable region of SEQ ID NO: 71 and a light chain variable region of SEQ ID NO: 72 (e.g., HVEM039);
10) an antibody including a heavy chain variable region of SEQ ID NO: 79 and a light chain variable region of SEQ ID NO: 80 (e.g., HVEM040); and
11) an antibody including a heavy chain variable region of SEQ ID NO: 87 and a light chain variable region of SEQ ID NO: 88 (e.g., HVEM041).

In an embodiment, the antibody may bind to the same epitope as any one of HVEM011, HVEM021, HVEM004, HVEM017, HVEM018, HVEM032, HVEM039, and HVEM040.

In an embodiment, the antibody may be any one of HVEM011, HVEM021, HVEM001, HVEM004, HVEM017, HVEM018, HVEM030, HVEM032, HVEM039, HVEM040, and HVEM041.

In an embodiment, the antibody may be any one of HVEM011, HVEM021, HVEM004, HVEM017, HVEM018, HVEM032, HVEM039, and HVEM040.

In an embodiment, the antibody may be a human antibody or a humanized antibody.

In an embodiment, the antibody may be a chimeric antibody.

Amino acid sequence variants of an antibody fall within the scope of the present disclosure so long as they improve or maintain or at least do not impair the desired properties. For example, the variants may include deletion, insertion, and/or substitution mutations of residues in the amino acid sequence of the antibody. Mutations may occur in HVRs and/or FRs. Substitutions may include conservative and non-conservative substitutions. Examples of conservative substitutions are shown in Table 1.

**[Table 1]**

| Residue | Exemplary substitution |
|---|---|
| Ala(A) | Val, Leu, Ile |
| Arg(R) | Lys, Gln, Asn |
| Asn(N) | Gln, His, Asp, Lys, Arg |
| Asp(D) | Glu, Asn |
| Cys(C) | Ser, Ala |
| Gln(Q) | Asn, Glu |
| Glu(E) | Asp, Gin |
| Gly(G) | Ala |
| His(H) | Asn, Gln, Lys, Arg |
| Ile(I) | Leu, Val, Met, Ala, Phe, Norleucine |
| Leu(L) | Norleucine, Ile, Val, Met, Ala, Phe |
| Lys(K) | Arg, Gln, Asn |
| Met(M) | Leu, Phe, Ile |
| Phe(F) | Trp, Leu, Val, Ile, Ala, Tyr |
| Pro(P) | Ala |
| Ser(S) | Thr |
| Thr(T) | Val, Ser |
| Trp(W) | Tyr, Phe |
| Tyr(Y) | Trp, Phe, Thr, Ser |
| Val(V) | Ile, Leu, Met, Phe, Ala, Norleucine |

Amino acids may be classified according to common side chain properties as follows:
1) hydrophobic: Norleucine, Met, Ala, Val, Leu, Ile;
2) neutral hydrophilic: Cys, Ser, Thr, Asn, Gln;
3) acidic: Asp, Glu;
4) basic: His, Lys, Arg;
5) residues that affect chain orientation: Gly, Pro; and
6) aromatic: Trp, Tyr, Phe.

Non-conservative substitutions may involve exchanging a member of one of the above groups for another member of the same group.

In addition, glycosylation variants, Fc region variants and cysteine engineered variants of the antibody, and antibody derivatives containing additional nonproteinaceous moieties (e.g., water-soluble polymers including polyethylene glycol) also fall within the scope of the present disclosure.

According to another aspect of the present disclosure, an isolated nucleic acid encoding the anti-HVEM antibody is provided. The nucleic acid may encode an amino acid sequence containing the VL of the antibody and/or an amino acid sequence containing the VH of the antibody.

According to another aspect of the present disclosure, a host cell including the nucleic acid is provided. The host cell may include: a vector including a nucleic acid that encodes an amino acid sequence containing the VL of the antibody and an amino acid sequence containing the VH of the antibody; or a first vector including a nucleic acid that encodes an amino acid sequence containing the VL of the antibody and a second vector including a nucleic acid that encodes an amino acid sequence containing the VH of the antibody. For example, the host cell may be a eukaryotic cell, such as a Chinese Hamster Ovary (CHO) cell.

According to another aspect of the present disclosure, provided is a method of producing an anti-HVEM antibody, including: culturing a host cell including a nucleic acid encoding the antibody under conditions suitable for expression of the antibody; and optionally recovering the antibody from the host cell or a culture medium thereof.

### Pharmaceutical Composition, Use, and Method

According to another aspect of the present disclosure, provided is a pharmaceutical composition for treating cancer, including the antibody as an active ingredient and a pharmaceutically acceptable carrier.

The pharmaceutically acceptable carrier enhances or stabilizes the composition or facilitates the preparation of the composition. The pharmaceutically acceptable carrier includes a physiologically compatible solvent, a dispersion medium, a coating agent, antibacterial and antifungal agents, an isotonic agent, and an absorption delaying agent, and the like.

The pharmaceutical composition of the present disclosure may be administered by various methods known in the art. Administration routes and/or methods vary depending on the desired results. Administration may be intravenous administration, intramuscular administration, intraperitoneal administration, or subcutaneous administration, or may be administration in the vicinity of a target site. The pharmaceutically acceptable carrier should be suitable for intravenous, intramuscular, subcutaneous, parenteral, spinal or epidermal administration (e.g., by injection or infusion). The composition should be sterile and fluid. The composition may be in lyophilized form. Isotonic agents such as sugars, polyalcohols such as mannitol or sorbitol, and sodium chloride may be included in the composition. The pharmaceutical composition of the present disclosure may be prepared according to methods well known and commonly used in the art.

A dosage level of the active ingredient in the pharmaceutical composition may vary to obtain an effective amount of the active ingredient sufficient to achieve the desired therapeutic response, depending on a particular patient, composition and administration method, while not being toxic to the patient. The selected dosage level varies depending on a variety of pharmacokinetic factors, e.g., the particular composition of the present disclosure used, or administration route, administration time, excretion rate, duration of treatment, other drugs used in combination, compounds and/or substances, the age, gender, body weight, condition and general health and previous medical history of a patient to be treated, and other factors. As a non-limiting example, the dosage may be in a range of about 0.0001 mg/kg to about 100 mg/kg, more typically in a range of 0.1 mg/kg to 20 mg/kg. Exemplary therapies involve administration once a week, once every two weeks, once a month, or once every 3 to 6 months.

In an embodiment, the composition may be for treating solid tumor or hematologic malignancy. Non-limiting examples of the solid tumor include breast cancer, lung cancer, head or neck cancer, colorectal cancer, esophageal cancer, laryngeal cancer, gastric cancer, liver cancer, pancreatic cancer, bone cancer, skin cancer, skin or intraocular melanoma, uterine cancer, ovarian cancer, rectal cancer, proximal anal cancer, colon cancer, breast cancer, fallopian tube carcinoma, endometrial carcinoma, cervical carcinoma, vaginal carcinoma, vulvar carcinoma, Hodgkin's disease, small intestine cancer, endocrine cancer, thyroid cancer, parathyroid cancer, adrenal cancer, soft tissue sarcoma, urethral cancer, penile cancer, prostate cancer , lymphocytic lymphoma, bladder cancer, renal or ureteral cancer, renal cell carcinoma, renal pelvic carcinoma, CNS tumor, primary CNS lymphoma, spinal cord tumor, brainstem glioma, and pituitary adenoma. Non-limiting examples of the hematologic malignancy include: leukemias, including acute myeloid leukemia, acute lymphocytic leukemia, and multiple myeloma; and lymphoid cell neoplasms such as chronic lymphocytic leukemia (CLL), non-Hodgkin lymphoma (NHL), small lymphocytic lymphoma (SLL), and mantle cell lymphoma (MCL). Non-Hodgkin lymphoma (NHL) include B and T non-Hodgkin lymphoma. In addition, cell lymphoid neoplasms include B, NK and T cell lymphoid neoplasms.

In an embodiment, the composition may be for use in combination with one or more other therapeutic agents, e.g., anticancer agents, antiviral agents, cytokines, or an immune agonist.

In an embodiment, the composition may further include at least one anticancer agent. For example, the anticancer agent may be a chemotherapeutic agent or an immunotherapeutic agent. Non-limiting examples of the chemotherapeutic agent include alkylating agent, nitrosourea agent, antimetabolites, anticancer antibiotics, vegetable-origin alkaloids, topoisomerase inhibitors, hormone drugs, hormone antagonists, leucopenia (neutropenia) treatment drugs, thrombocytopenia treatment drugs, antiemetics, aromatase inhibitors, P-glycoprotein inhibitors, platinum complex derivatives, other immunotherapeutic drugs, and other anticancer drugs. Exemplary cytotoxic agents that may be administered in combination include antimicrotubule agents, topoisomerase inhibitors, anti-metabolites, mitotic inhibitors, alkylating agents, anthracyclines, *vinca* alkaloids, intercalating agents, agents capable of interfering with a signal transduction pathway, agents that promote apoptosis, proteasome inhibitors, and radiation (local or whole-body irradiation). Non-limiting examples of additional therapeutic agents include peptides, polypeptides, proteins, fusion proteins, nucleic acid molecules, small molecules, mimetic agents, synthetic drugs, inorganic molecules, and organic molecules.

The immunotherapeutic agent refers to a compound, composition or treatment that indirectly or directly enhances, stimulates or increase the body's immune response against cancer cells and/or that decreases the side effects of other anticancer therapies. Non-limiting examples of the immunotherapeutic agent include cytokines, cancer vaccines, monoclonal antibodies, non-cytokine adjuvants, immune cells (T cells, NK cells, dendritic cells, B cells, and the like), immune checkpoint inhibitors, and microorganisms. In an embodiment, the immunotherapeutic agent is an immune checkpoint inhibitor. The immune checkpoint inhibitor includes peptides, antibodies, nucleic acid molecules, and small molecules. For example, the immune checkpoint inhibitor may be administered to enhance the proliferative, migratory, persistent and/or cytotoxic activity of CD8+ T cells in a subject, and in particular, the tumor invasiveness of CD8+ T cells in a subject. Typically, immune checkpoint inhibitors are antagonists that block immunosuppressive receptors expressed by NK cells, or antagonists that block key ligands of these receptors, for example, PD-1 ligand CD274 (best known as PD-L1 or B7 H1), such as activated T lymphocytes, e.g., cytotoxic T lymphocyte-associated protein 4 (CTLA4) and programmed cell death 1 (PD-1), or various members of the killer cell immunoglobulin-like receptor (KIR) family. For example, the immune checkpoint inhibitor is an antibody or an antigen-binding fragment thereof. Specifically, the immune checkpoint inhibitor may be at least one selected from the group consisting of anti-PD-1 antibodies, anti-PD-L1 antibodies, anti-PD-L2 antibodies, anti-CTLA-4 antibodies, anti-TIM-3 antibodies, anti-LAG3 antibodies, anti-IDO1 antibodies, anti-TIGIT antibodies, anti-VISTA antibodies, anti-B7H3 antibodies, anti-B7H4 antibodies, anti-BTLA antibodies, anti-B7H6 antibodies, and antigen-binding fragments of these antibodies. More specifically, the immune checkpoint inhibitor may be, but is not limited to, at least one selected from ipilimumab (Yervoy^{®}, BMS/Ono), tremelimumab (AstraZeneca), atezolizumab (Tecentriq^{®}, Roche), nivolumab (Opdivo^{®}, BMS/Ono), pembrolizumab (Keytruda^{®}, MSD), avelumab (Bavencio^{®}, Pfizer/Merck, Germany), durvalumab (Imfinzi^{®}, AstraZeneca/Medimmune), and antigen-binding fragments thereof.

In an embodiment, the immunotherapeutic agent may be formulated and used in various forms suitable for each purpose of use according to a method commonly used in the art, for example, as an oral formulation such as lipid, suspension, powder, granules, tablets, capsules, pills, extract, emulsion, syrup, and aerosol, and a parenteral formulation such as an injection of a sterile injection solution. The immunotherapeutic agent may be administered orally or parenterally via a variety of routes, including intravenous administration, intraperitoneal administration, subcutaneous administration, intradermal administration, intramuscular administration, spinal administration, intrathecal administration, rectal local administration, or injection. A dosage may vary depending on the patient's body weight, age, gender, health condition, diet, administration time, administration method, administration period or interval, excretion rate, constitutional specificity, nature of the formulation, severity of the disease, and the like, and may be appropriately selected by those of ordinary skill in the art. For example, the dosage may be, but is not limited to, in a range of about 0.1 mg/kg to about 10,000 mg/kg, and administration may be performed once a day or several times a day in aliquots.

A third aspect of the present disclosure provides a method of treating cancer in a subject, including a step of administering a therapeutically effective amount of the antibody to a subject in need thereof.

Subjects to be administered may include humans or animals, such as humans, pigs, dogs, cats, cows, horses, mice, and the like without limitation.

Hereinafter, the present disclosure will be described in detail with reference to the following examples, and these examples are provided to aid in understanding of the present disclosure and not intended to limit the scope of the present disclosure in any way.

### Example 1: Antibody Screening

To construct an antibody binding to Herpes virus entry mediator (HVEM) (TNFRSF14), a phage display library was used. The phage displayed-scFv library is a library in which a single chain variable fragment (scFv) consisting of a human antibody sequence synthesized at the pill terminal of a filamentous bacteriophage is fused, and consists of a heavy chain variable region (VH) and a light chain variable region (VL) of a human antibody, wherein VL is a kappa light chain.

To select human antibodies having specific affinity for HVEM, panning was performed with two strategies. The first strategy was to perform 3 rounds of panning with human HVEM only, and the second strategy was to conduct 1^{st} and 2^{nd} rounds of panning with human HVEM and 3^{rd} round with mouse HVEM.

The phage pool obtained in each strategy was infected with *E. coli* to form a single colony, and a monoclonal enzyme-linked immunosorbent assay (ELISA) was performed. A single colony was cultured in Super Broth (SB) medium containing carbenicillin at 37 °C until OD reached 0.5, and then 1 mM isopropyl β-D-1-thiogalactopyranoside (IPTG) was added, followed by incubation overnight at 30 °C. The supernatant was removed by centrifugation, and the pellet was resuspended in TES buffer to obtain a periplasmic extract. The obtained periplasmic extract was mixed 1:1 with blocking buffer and used for ELISA, and the antibody bound to HVEM was detected through the hemagglutinin (HA) tag. Positive clones were set as clones having an OD of 0.2 or more and an OD of 5 times or more the background. Through strategy 1, 73 antibodies that bind to human HVEM were selected, and 34 of them were randomly selected to confirm cross-reactivity with mouse HVEM through ELISA. 31 out of 34 showed cross-reactivity, except for three. Through strategy 2, 2 clones specific to human HVEM and 57 clones showing cross-reactivity with human and mouse HVEM were selected. Through DNA sequencing of the selected clones, 50 individual clones showing cross-reactivity with human and mouse HVEM and having different nucleotide sequences were obtained.

### Example 2: Purification of scFv Antibody and Verification of HVEM Antigen Binding Ability

The antigen binding ability of the obtained HVEM antibody was examined through ELISA. The selected clones were transformed into HB2151 (non-suppressor *E. coli*) to purify scFv antibodies. Transformed HB2151 was cultured in SB medium (+ carbenicillin) at 37 °C until OD reached 0.5, and IPTG was added to a final concentration of 1 mM, followed by incubation overnight at 30 °C. The culture medium was centrifuged to remove the supernatant and a pellet is obtained. The obtained pellet was resuspended in TES buffer and the supernatant was obtained by centrifugation.

For purification of scFv from the periplasmic extract containing the scFv, Ni-NTA purification was performed using a 6×his tag included at the end. Through purification, 44 scFvs out of 50 were obtained.

Using the purified antibodies, binding ability (affinity) for each of the human HVEM and mouse HVEM antigens was measured. ELISA was used as a measurement method. 33 types of antibodies with a binding ability to a human HVEM antigen of 10 nM or less and a binding ability to a mouse HVEM antigen of 50 nM or less were selected. The selected clones and the binding abilities thereof are shown in Table 2.

**[Table 2]**

| scFv | Affinity (nM) for Human HVEM (nM) | Affinity (nM) for Mouse HVEM (nM) |
|---|---|---|
| P1SH001 | 0.2 | 3.9 |
| P1SH002 | 1.2 | 25.4 |
| P1SH004 | 0.3 | 0.4 |
| P1SH005 | 0.3 | 1.2 |
| P1SH006 | 0.9 | 3.7 |
| P1SH007 | 0.4 | 3 |
| P1SH008 | 1 | 1.6 |
| P1SH009 | 0.3 | 6.1 |
| P1SH011 | 0.4 | 1 |
| P1SH012 | 0.4 | 1.4 |
| P1SH014 | 0.5 | 12.7 |
| P1SH015 | 0.4 | 48 |
| P1SH017 | 0.9 | 4.1 |
| P1SH018 | 1.7 | 4.6 |
| P1SH020 | 1.8 | 7.2 |
| P1SH021 | 3.8 | 14 |
| P1SH022 | 0.3 | 7.5 |
| P1SH023 | 0.08 | 0.3 |
| P1SH026 | 1 | 1.5 |
| P1SH029 | 1.6 | 50.5 |
| P1SH030 | 1.2 | 3.3 |
| P1SH032 | 9.1 | 26.9 |
| P1SH033 | 10 | 24.3 |
| P1SH035 | 3 | 13.8 |
| P1SH039 | 0.5 | 29.3 |
| P1SH040 | 0.08 | 0.3 |
| P1SH041 | 0.5 | 2.8 |
| P1SH042 | 0.06 | 0.3 |
| P1SH043 | 0.05 | 0.1 |
| P1SH046 | 0.4 | 1.2 |
| P1SH048 | 6.8 | 3.1 |
| P1SH049 | 0.1 | 0.2 |
| P1SH050 | 3.6 | 15.9 |

### Example 3: Construction of Full-Size Human Antibodies

A full-size human antibody was constructed using the VH and VL of the selected scFv antibody and the heavy chain constant region (CH) and light chain constant region (CL) of a human antibody.

To construct the heavy chain (HC) of the full-size human antibody, a signal sequence, the VH region of the selected scFv antibody, and the CH of human antibody IgG4 were fused. The signal peptide of human IL-2 was used for the high expression of the antibody and release thereof into a medium. The CH of human antibody IgG4 was based on the natural heavy chain CH, and serine 228 in the hinge region, on the basis of the EU numbering system, was substituted with proline.

To construct the light chain (LC) of the full-size human antibody, a signal sequence, the VL region of the selected scFv antibody, and human antibody CL were fused. The signal peptide of human IL-2 was used for the high expression of the antibody and release thereof into a medium. CL of a natural kappa light chain was used as CL of the human antibody light chain. Amino acid sequences of CH and CL fused to construct the corresponding antibody are represented as SEQ ID NO: 89 and SEQ ID NO: 90, respectively.

To construct a vector expressing the antibody, a cassette vector was constructed by cloning the human IL-2 signal peptide, CH, and CL. VH and VL of the antibody were amplified from scFv antibody sequences by PCR and used for cloning. To construct a vector expressing the heavy chain of the antibody, a heavy chain cassette vector was cleaved with Afel restriction enzyme, and the amplified VH was cloned using a PCR method. To construct a vector expressing the light chain of the antibody, a kappa light chain cassette vector was cleaved with Afel and BsiWI restriction enzymes, and the amplified VL was cloned using a PCR method. For the HVEM011 and HVEM021 antibodies, DNA sequences subjected to CHO cell codon optimization were synthesized and then amplified by PCR and used for cloning.

For the production of antibody proteins, a vector expressing the heavy chain (HC) of each antibody and a vector expressing the light chain (LC) of each antibody were used. An ExpiCHO-S cell line was co-transfected with the two vectors (HC, LC) expressing each antibody to induce expression of the antibody. A culture medium from which suspended matter containing cells was removed was purified using MabselectSure and Hitrap SP FF purification columns. The purified proteins were subjected to purity analysis through SEC-HPLC and endotoxin analysis, and then was used for *in vitro* testing, cell-based testing, and animal testing.

### Example 4: Analysis of Antibody/Antigen Binding Ability

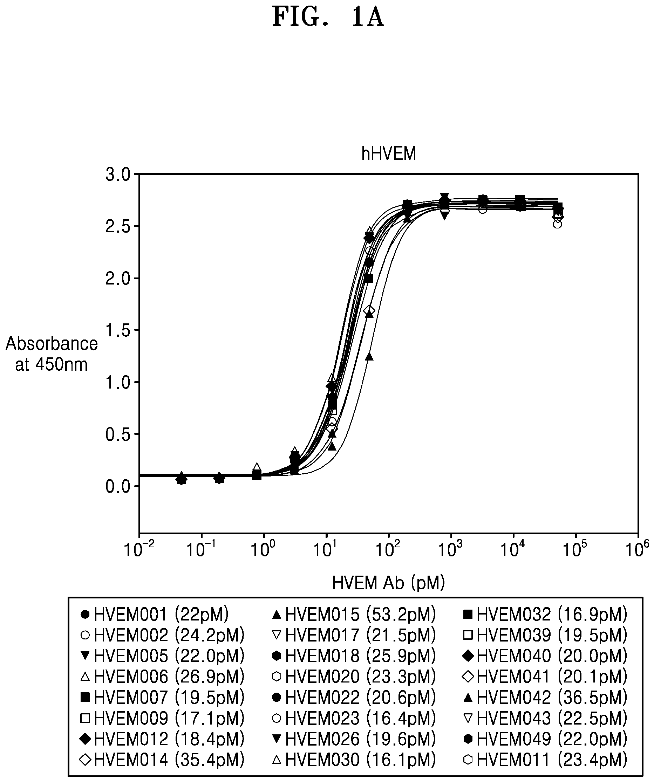

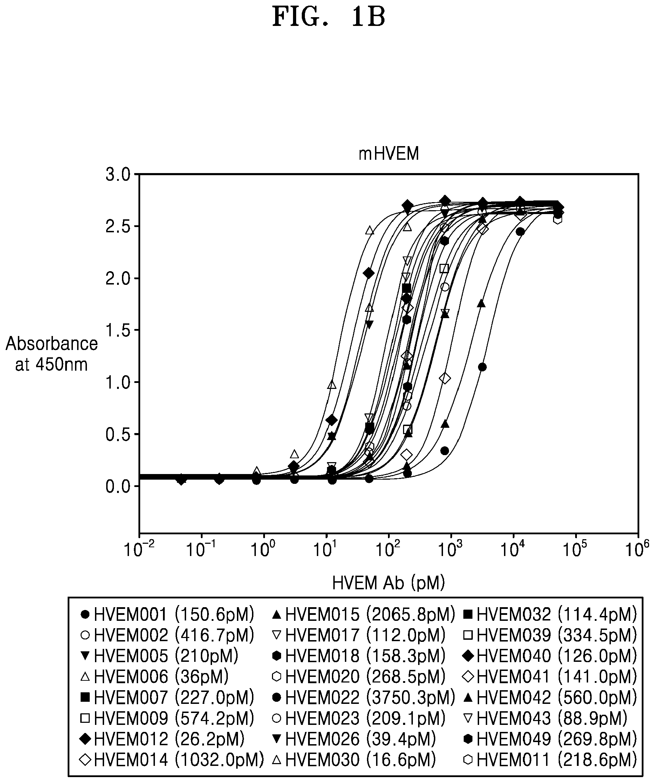

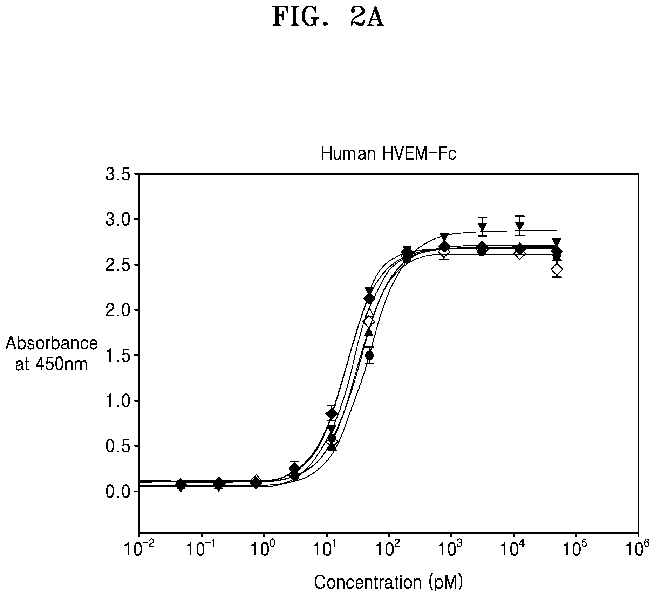

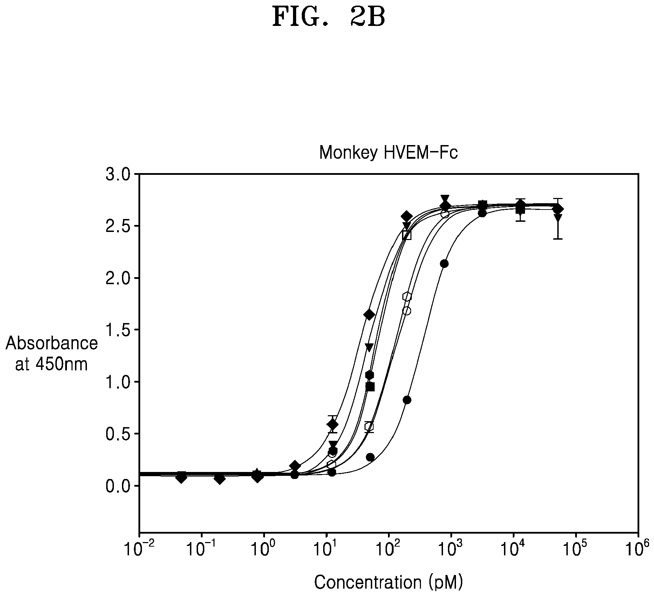

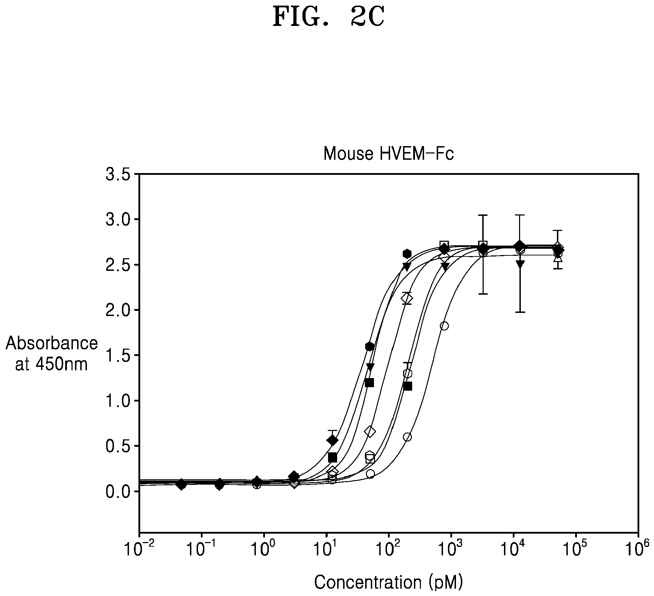

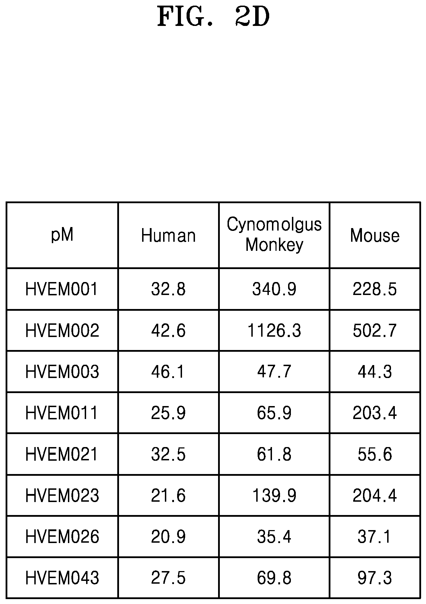

The binding ability of the full-size human antibody to HVEM was analyzed using an ELISA testing method. 0.2 µg/ml of each of human HVEM-hFc, cynomolgus monkey HVEM-hFc, and mouse HVEM-mFc proteins was coated on a 96-half-well plate for immunosorbent assay. To inhibit non-specific binding, reaction was carried out in phosphate buffered saline (PBS) containing 3% bovine serum albumin (BSA) for 1 hour, followed by treatment with serially diluted antibodies for 1 hour. To measure the amounts of the bound antibodies, horse radish peroxidase (HRP)-conjugated anti-human IgG4 antibody was used, and for the activity of HRP, a color reaction was induced using a 3,3',5,5'-tetramethylbenzidine (TMB) substrate solution, and the reaction was terminated with 1N sulfuric acid. The degree of color reaction was confirmed using SpectraMax i3 (Molecular Devices, 10192-220). In the middle of each step, washing was performed three times using PBS (PBST) containing 0.1% Triton X-100. The binding ability of the antibodies was derived using SigmaPlot. The binding affinity of the anti-HVEM antibodies to human HVEM is illustrated in FIG. 1A, and the binding affinity of the anti-HVEM antibodies to mouse HVEM is illustrated in FIG. 1B. The results of cross-reactivity of the anti-HVEM antibodies with human, cynomolgus monkey and mouse HVEM are illustrated in FIGS. 2A, 2B, 2C, and 2D.

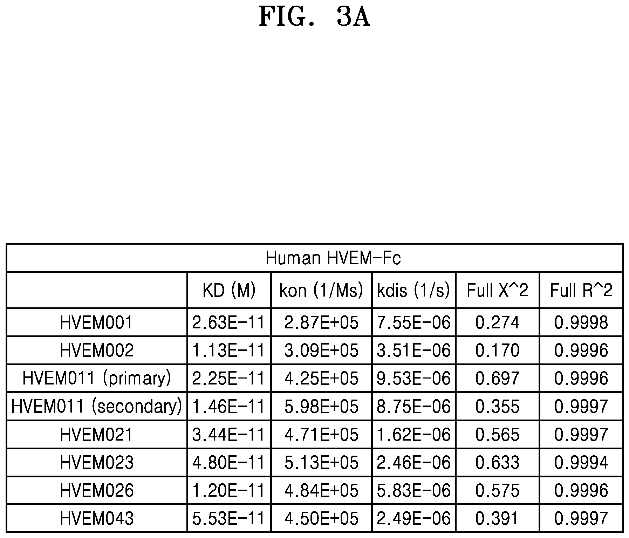

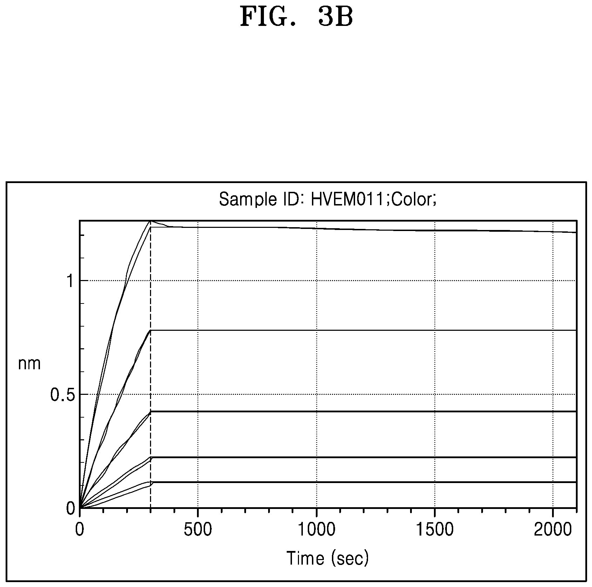

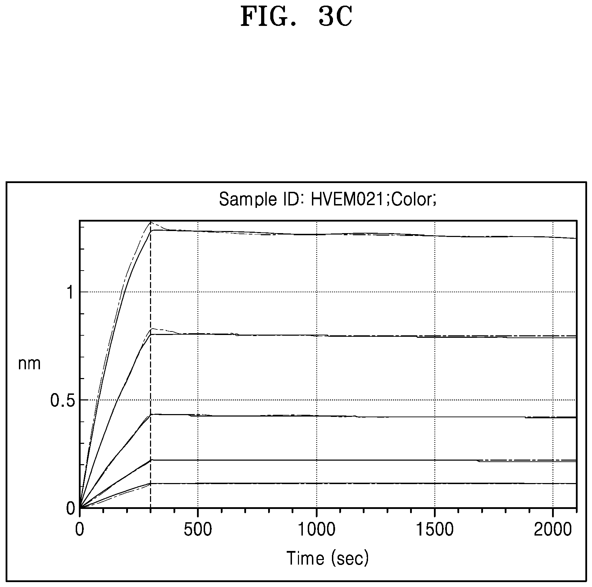

Binding ability to human HVEM antigen was further measured using an Octet BLI assay. The binding ability of the HVEM antibodies to human HVEM antigen as measured using Octet BLI is illustrated in FIGS. 3A, 3B (HVEM011), and 3C (HVEM021).

### Example 5: Identification of Epitope by Antigen/Antibody Competitive Binding

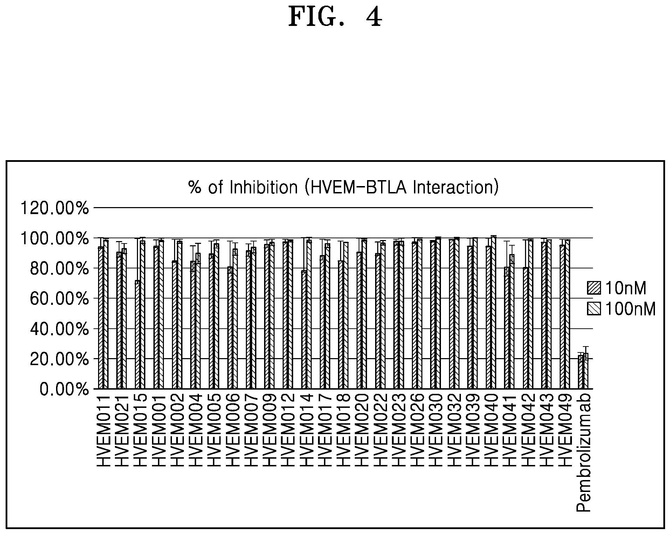

To measure competitive binding of HVEM antibodies, a cell-based assay was performed. To measure the HVEM competitive binding between HVEM antibodies and BTLA protein, the BTLA protein was stably expressed in a Jurkat-Lucia^{™} NFAT (InvivoGen) cell line and used. 5×10⁵ cells were treated with 1 µg/ml of human HVEM-Fc protein and 10 nM or 100 nM of each HVEM antibody and a reaction was allowed to occur on ice for 2 hours. To measure human HVEM-Fc bound to the cell line, Alexa488-conjugated anti-human antibody IgG1 (Invitrogen A10631) was used. For flow cytometry, the stained cell lines were fixed on ice for 20 minutes using PBS containing 2% formaldehyde and stored in a refrigerator until flow cytometry. Flow cytometry was performed using a FACSVerse^{™} flow cytometer (BD Biosciences), and the amount of bound human HVEM-Fc was analyzed using the FlowJo_v10 program. The results of the corresponding cases are illustrated in FIG. 4, and all of the HVEM antibodies used were observed to have competitive binding with BTLA for HVEM on the surface of the cells.

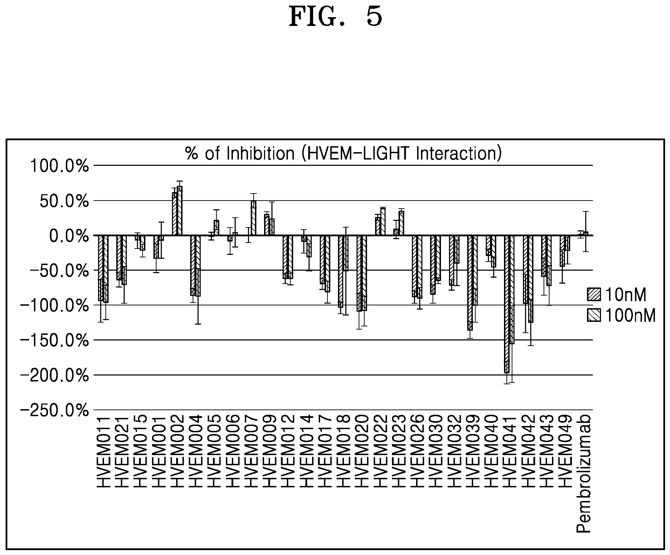

To measure the HVEM competitive binding between HVEM antibodies and a LIGHT protein, the HVEM protein was stably expressed in a Jurkat-Lucia^{™} NFAT (InvivoGen) cell line and used. 5×10⁵ cells were simultaneously treated with 0.25 µg/ml of human LIGHT protein and 10 nM or 100 nM of each HVEM antibody, and a reaction was allowed to occur on ice for 2 hours. To measure the human LIGHT protein bound to the cell line, allophycocyanin (APC)-conjugated anti-His antibody (Abcam ab72579) was used. For flow cytometry, the stained cell lines were fixed using PBS containing 2% formaldehyde and stored in a refrigerator until flow cytometry. Flow cytometry was performed using a FACSVerse^{™} flow cytometer (BD Biosciences), and the amount of bound human LIGHT protein was analyzed using the FlowJo_v10 program. The results thereof are illustrated in FIG. 5. Only some antibodies, such as HVEM002 antibody, were observed to have competitive binding with the LIGHT protein, and antibodies, including HVEM011 and HVEM021, were observed to exhibit increased binding to LIGHT.

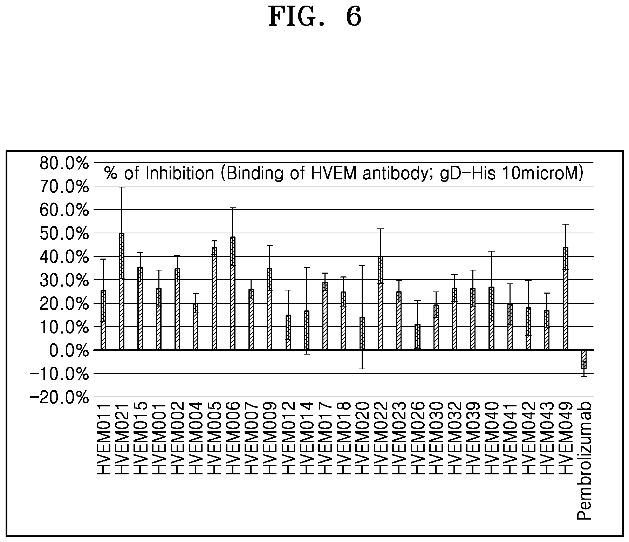

To measure the HVEM competitive binding between the HVEM antibodies and herpes virus glycoprotein D (gD), the HVEM protein was stably expressed in a Jurkat-Lucia^{™} NFAT (InvivoGen) cell line and used. 5×10⁵ cells were reacted with 10 µM of gD protein for 2 hours, and 1 nM of each HVEM antibody was added thereto and a reaction was allowed to occur for 1 hour. To measure the HVEM antibodies bound to the cell line, fluorescein isothiocyanate (FITC)-conjugated anti-human antibody IgG4 (Abcam ab99821) was used. For flow cytometry, the stained cell lines were fixed using PBS containing 2% formaldehyde and stored in a refrigerator until flow cytometry. Flow cytometry was perforemd using a FACSVerse^{™} flow cytometer (BD Biosciences), and the amounts of bound HVEM antibodies were analyzed using the FlowJo_v10 program. The results thereof are illustrated in FIG. 6. Most of the HVEM antibodies used in the test showed competitive binding to herpes virus glycoprotein D and HVEM on the surface of the cells.

### Example 6: HVEM Immune Checkpoint Inhibitory Ability

To measure the HVEM-BTLA immune checkpoint inhibitory ability of the HVEM antibodies, a cell-based assay system in which the HVEM-BTLA immune checkpoint works was constructed. First, to produce T cells expressing BTLA, BTLA was stably expressed in a Jurkat-Lucia^{™} NFAT (InvivoGen) cell line. To this end, the infection was made using a lentivirus expressing human BTLA, and under conditions in which BTLA expression was stably maintained in cells by 90% or more, infected Jurkat-Lucia^{™} NFAT cell line was used. This cell line was named Jurkat-luciaNFAT-hBTLA.

To activate the Jurkat-luciaNFAT-hBTLA cell line, 96-well cell culture plates were coated overnight with 50 µl of an anti-CD3 agonist antibody (OKT3) diluted to 5 µg/ml. To induce an immunosuppressive response by the HVEM-BTLA immune checkpoint, human HVEM-Fc protein was treated with a cell culture medium. 40 nM of human HVEM-Fc and 5×10⁵ cells were mixed, followed by culture in 100 µl of culture medium for 24 hours on OKT3 antibody-coated plates. When the HVEM-BTLA immune checkpoint inhibitory ability of the antibodies was measured, the HVEM antibodies and an isotype control antibody were used, and the culture was performed after adding the antibodies together with human HVEM-Fc and the Jurkat-luciaNFAT-hBTLA cell line. After 24 hours of reaction, the culture medium was collected, and the activity of Lucia luciferase secreted in the culture medium was measured using QUANTI-Luc^{™} (InvivoGen) reagent. 10 µl of the culture medium was mixed with 50 µl of a QUANTI-Luc^{™} reagent, and then luminescence was measured using a luminometer.

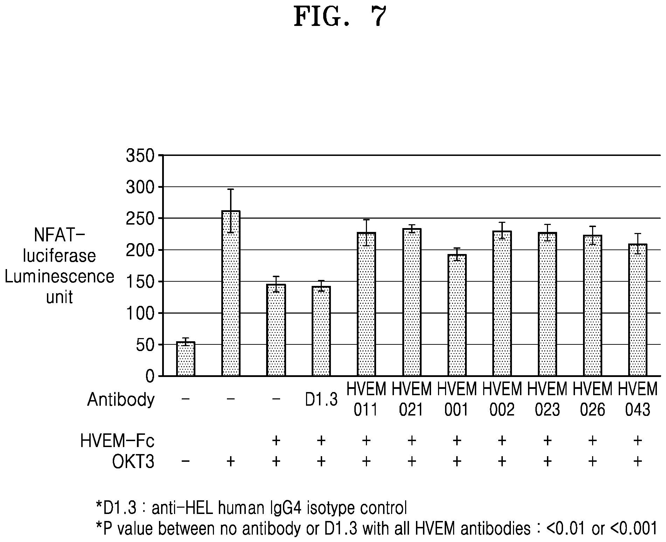

The results thereof are illustrated in FIG. 7. In this experiment, the Jurkat-luciaNFAT-hBTLA cell line was observed to have low luciferase activity since the immune checkpoint was activated when treated with human HVEM-Fc protein, whereas the Jurkat-luciaNFAT-hBTLA cell line was observed to have restored luciferase activity since the immune checkpoint was inactivated when treated with HVEM antibodies.

### Example 7: Verification of Anticancer Efficacy in Animal Models

To verify the anti-tumor animal efficacy of the corresponding HVEM antibody, the following model was used.

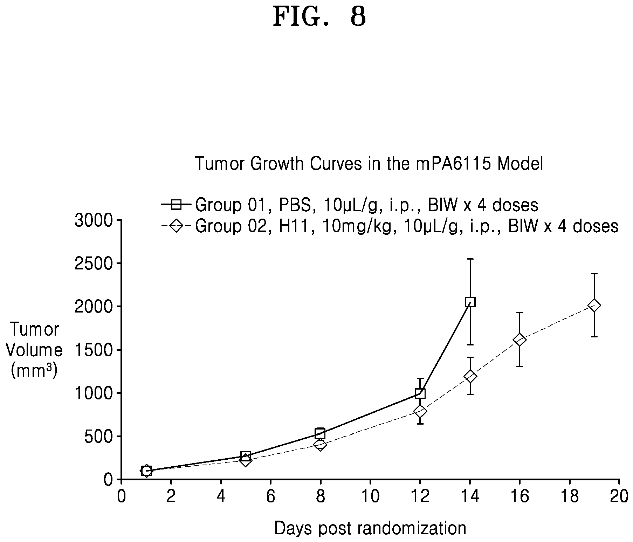

To verify efficacy in a pancreatic cancer model, in the case of Pdx1-Cre; Kras(G12D/+); Trp53-/-(KPC) mPA6115 model, which is a mouse homograft model, the volume of cancer tissue in HVEM antibody-administered groups was measured and compared with a vehicle (PBS) control. Cancer tissue having a diameter of 2 mm to 3 mm was subcutaneously implanted to construct a model. Administration was started when the volume of the cancer tissue reached about 100 mm³. Administration was carried out twice a week, a total of 4 times, and the amount of antibody administered was 10 mg/kg based on the body weight of mice, and the route of the administration was intraperitoneal administration. The volume of cancer tissue is measured by using a caliper to measure the maximum length (L) of the cancer tissue and the width (W) for the maximum length of the cancer tissue, and calculated by Equation: (V=L×W×W/2). The results thereof are illustrated in FIG. 8. When measured 14 days after group separation and first administration, the inhibition rate of cancer growth compared to a negative control was 41.69%.

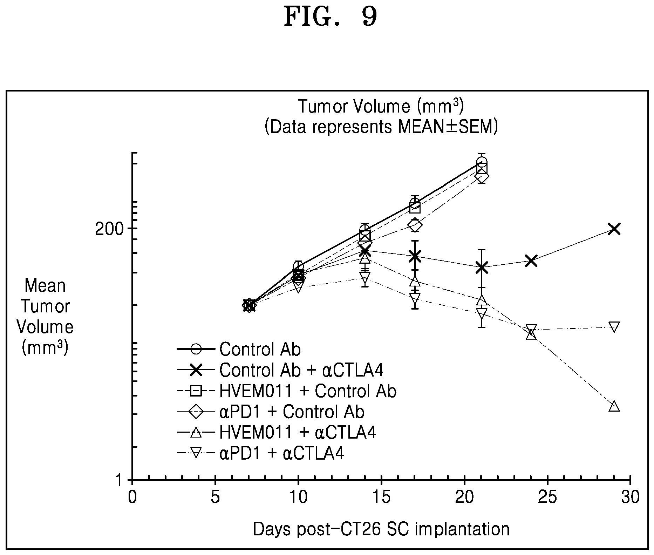

To investigate the efficacy of combined use of anti-CTLA-4 and anti-PD-1 antibodies, a CT-26 mouse model was used. 5×10⁵ CT-26 cells were transplanted subcutaneously, and administration was started after group separation when the volume of cancer tissue reached 50 mm³. 10 mg/kg of a HVEM antibody, 10 mg/kg of RMP1-14 anti-PD-1 antibody (BioXCell), and 5 mg/kg of 9H10 anti-CTLA-4 antibody (BioXCell) were administered alone or in combination, and an isotype control antibody was used as a negative control for the HVEM antibody. The antibodies were administered twice a week, a total of 4 times. The results thereof are illustrated in FIG. 9. The group administered with 9H10 anti-CTLA-4 antibody alone and the groups administered with the antibodies in combination exhibited a high inhibition rate of tumor growth, and the combined use of anti-PD-1 and HVEM antibodies exhibited an increased inhibition rate of tumor growth compared to when the 9H10 anti-CTLA-4 antibody was administered alone. A significant increase in the tumor growth inhibition rate was observed in the administration in combination with the HVEM antibody compared to the administration in combination with anti-PD-1 (on the basis of 29 days after cancer cell transplantation, and 23 days after the first administration).

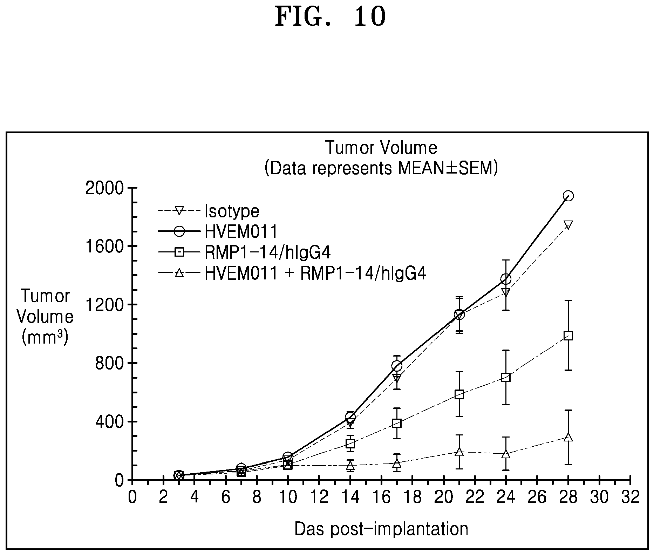

A cell line in which the expression of mouse HVEM was stably increased in the CT-26 cancer cell line was constructed, and a mouse model was constructed using the cell line. The CT-26 cell line was transformed with a vector expressing mouse HVEM, and then cells in which mouse HVEM was highly and stably expressed were isolated to construct a cell line, which was denoted as CT-26-mHVEM#2-2. 5 × 10⁵ CT-26-mHVEM#2-2 cells were subcutaneously transplanted, and antibody administration was initiated 3 days later, and each antibody was administered intraperitoneally at a dose of 10 mg/kg twice a week, a total of 5 times. The isotype control was used as a negative control, and RMP1-14/hlgG4 was co-administered. RMP1-14/hlgG4 is a chimeric antibody in which the variable region of an anti-PD-1 RMP1 -14 antibody and the constant region of a human antibody are fused. The results thereof are illustrated in FIG. 10. A significant increase in the tumor growth inhibition rate was observed upon combined administration of the HVEM antibody and the anti-PD-1 antibody, compared to the group treated with the anti-PD-1 antibody alone.

### Example 8: Verification of Anticancer Efficacy in Humanized Animal Models

A humanized mouse model was used to investigate anticancer efficacy on human immune cells and cancer cells. To produce humanized cancer model mice, NSGA immunodeficient mice, SNU407 or PC3 human cancer cell line, and peripheral blood mononuclear cells (PBMCs) isolated from human blood were used. It was confirmed that, in the SNU407 human cancer cell line, HVEM was highly expressed and very low expression of PD-L1 was observed. It was confirmed that expression of both HVEM and PD-L1 was observed in the PC3 human cancer cell line.

Human cancer tissue was created by transplanting the human cancer cell line SNU407 into NSGA immunodeficient mice, and 3 days later, human immune cells were induced to settle in the mice by transplanting PBMCs isolated from human blood. Antibody administration was started one week after PBMC transplantation, and each antibody was intraperitoneally administered at a dose of 10 mg/kg twice a week, a total of five times. The isotype control was used as a negative control, and atezolizumab was used as a control for antibodies binding to PD-L1 of transplanted human cancer cells. Two types of HVEM antibodies were compared and verified.

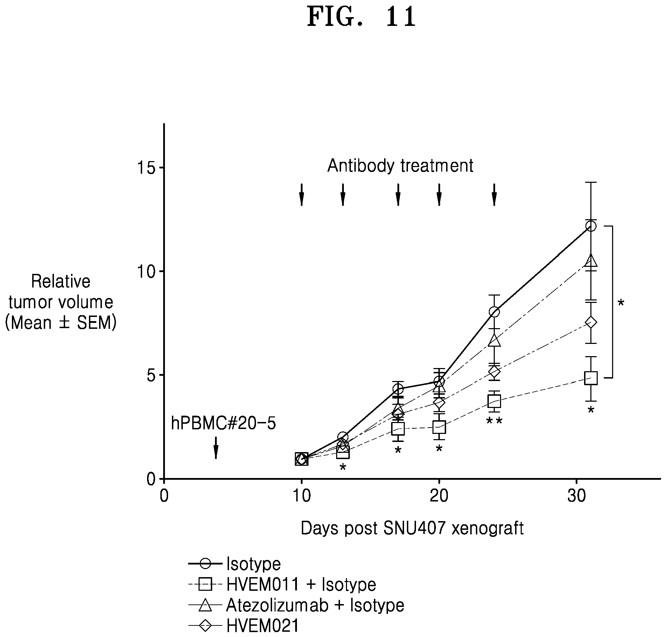

The results thereof are illustrated in FIG. 11. It was confirmed that the tumor growth inhibitory effect of the HVEM antibodies was higher in the SNU407 model with high HVEM expression than in atezolizumab.

Human cancer cell line PC3 was transplanted into NSGA immunodeficient mice to make human cancer tissues, and 3 days later, human PBMCs were transplanted to induce human immune cells to settle in the mice. Antibody administration was started 4 days after PBMC transplantation, each antibody was administered at 10 mg/kg, and a total of 20 mg/kg of the antibody mixture was administered on the basis of combined administration. Each antibody was intraperitoneally administered twice a week, a total of 11 times.

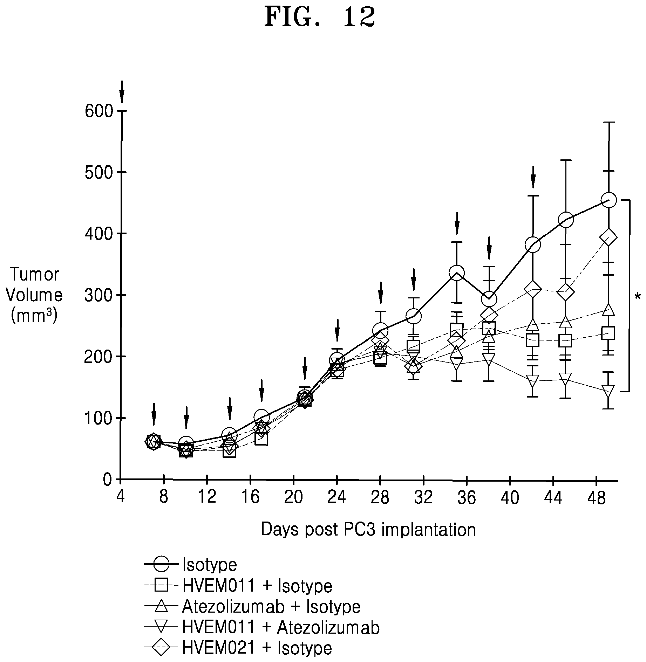

The results thereof are illustrated in FIG. 12. It was confirmed that, in the PC3 model in which both HVEM expression and PD-L1 expression were observed, the combined administration of the HVEM antibody and the PD-L1 antibody exhibited an increased tumor growth inhibitory effect, compared to the single administration.

## Claims

1. An anti-HVEM monoclonal antibody that binds to human Herpes Virus Entry Mediator (HVEM) with a dissociation constant (Kd) of 500 pM or less, inhibits binding of HVEM to B and T lymphocyte attenuator (BTLA) and promotes binding of HVEM to LIGHT.

2. The anti-HVEM monoclonal antibody of claim 1, wherein the anti-HVEM monoclonal antibody binds to human HVEM with a dissociation constant (Kd) of 50 pM or less.

3. The anti-HVEM monoclonal antibody of claim 1 or 2, wherein the anti-HVEM monoclonal antibody inhibits binding of HVEM to gD.

4. The anti-HVEM monoclonal antibody of any one of claims 1 to 3, wherein the anti-HVEM monoclonal antibody binds to the same epitope as any one of the following antibodies:
1) an antibody comprising HCDR1 of SEQ ID NO: 1, HCDR2 of SEQ ID NO: 2, HCDR3 of SEQ ID NO: 3, LCDR1 of SEQ ID NO: 4, LCDR2 of SEQ ID NO: 5, and LCDR3 of SEQ ID NO: 6;
2) an antibody comprising HCDR1 of SEQ ID NO: 9, HCDR2 of SEQ ID NO: 10, HCDR3 of SEQ ID NO: 11, LCDR1 of SEQ ID NO: 12, LCDR2 of SEQ ID NO: 13, and LCDR3 of SEQ ID NO: 14;
3) an antibody comprising HCDR1 of SEQ ID NO: 17, HCDR2 of SEQ ID NO: 18, HCDR3 of SEQ ID NO: 19, LCDR1 of SEQ ID NO: 20, LCDR2 of SEQ ID NO: 21, and LCDR3 of SEQ ID NO: 22;
4) an antibody comprising HCDR1 of SEQ ID NO: 25, HCDR2 of SEQ ID NO: 26, HCDR3 of SEQ ID NO: 27, LCDR1 of SEQ ID NO: 28, LCDR2 of SEQ ID NO: 29, and LCDR3 of SEQ ID NO: 30;
5) an antibody comprising HCDR1 of SEQ ID NO: 33, HCDR2 of SEQ ID NO: 34, HCDR3 of SEQ ID NO: 35, LCDR1 of SEQ ID NO: 36, LCDR2 of SEQ ID NO: 37, and LCDR3 of SEQ ID NO: 38;
6) an antibody comprising HCDR1 of SEQ ID NO: 41, HCDR2 of SEQ ID NO: 42, HCDR3 of SEQ ID NO: 43, LCDR1 of SEQ ID NO: 44, LCDR2 of SEQ ID NO: 45, and LCDR3 of SEQ ID NO: 46;
7) an antibody comprising HCDR1 of SEQ ID NO: 49, HCDR2 of SEQ ID NO: 50, HCDR3 of SEQ ID NO: 51, LCDR1 of SEQ ID NO: 52, LCDR2 of SEQ ID NO: 53, and LCDR3 of SEQ ID NO: 54;
8) an antibody comprising HCDR1 of SEQ ID NO: 57, HCDR2 of SEQ ID NO: 58, HCDR3 of SEQ ID NO: 59, LCDR1 of SEQ ID NO: 60, LCDR2 of SEQ ID NO: 61, and LCDR3 of SEQ ID NO: 62;
9) an antibody comprising HCDR1 of SEQ ID NO: 65, HCDR2 of SEQ ID NO: 66, HCDR3 of SEQ ID NO: 67, LCDR1 of SEQ ID NO: 68, LCDR2 of SEQ ID NO: 69, and LCDR3 of SEQ ID NO: 70;
10) an antibody comprising HCDR1 of SEQ ID NO: 73, HCDR2 of SEQ ID NO: 74, HCDR3 of SEQ ID NO: 75, LCDR1 of SEQ ID NO: 76, LCDR2 of SEQ ID NO: 77, and LCDR3 of SEQ ID NO: 78; and,
11) an antibody comprising HCDR1 of SEQ ID NO: 81, HCDR2 of SEQ ID NO: 82, HCDR3 of SEQ ID NO: 83, LCDR1 of SEQ ID NO: 84, LCDR2 of SEQ ID NO: 85, and LCDR3 of SEQ ID NO: 86.

5. The anti-HVEM monoclonal antibody of claim 4, wherein anti-HVEM monoclonal antibody binds to the same epitope as any one of the following antibodies:
1) an antibody comprising a heavy chain variable region of SEQ ID NO: 7 and a light chain variable region of SEQ ID NO: 8;
2) an antibody comprising a heavy chain variable region of SEQ ID NO: 15 and a light chain variable region of SEQ ID NO: 16;
3) an antibody comprising a heavy chain variable region of SEQ ID NO: 23 and a light chain variable region of SEQ ID NO: 24;
4) an antibody comprising a heavy chain variable region of SEQ ID NO: 31 and a light chain variable region of SEQ ID NO: 32;
5) an antibody comprising a heavy chain variable region of SEQ ID NO: 39 and a light chain variable region of SEQ ID NO: 40;
6) an antibody comprising a heavy chain variable region of SEQ ID NO: 47 and a light chain variable region of SEQ ID NO: 48;
7) an antibody comprising a heavy chain variable region of SEQ ID NO: 55 and a light chain variable region of SEQ ID NO: 56;
8) an antibody comprising a heavy chain variable region of SEQ ID NO: 63 and a light chain variable region of SEQ ID NO: 64;
9) an antibody comprising a heavy chain variable region of SEQ ID NO: 71 and a light chain variable region of SEQ ID NO: 72;
10) an antibody comprising a heavy chain variable region of SEQ ID NO: 79 and a light chain variable region of SEQ ID NO: 80; and,
11) an antibody comprising a heavy chain variable region of SEQ ID NO: 87 and a light chain variable region of SEQ ID NO: 88.

6. The anti-HVEM monoclonal antibody of any one of claims 1 to 5, wherein the anti-HVEM monoclonal antibody is a human antibody or a humanized antibody.

7. The anti-HVEM monoclonal antibody of any one of claims 1 to 5, wherein anti-HVEM monoclonal antibody is a chimeric antibody.

8. An isolated nucleic acid encoding the antibody of any one of claims 1 to 7.

9. A host cell comprising the nucleic acid of claim 8.

10. A method of producing an antibody, comprising culturing the host cell of claim 9 under conditions suitable for expression of a nucleic acid encoding an anti-HVEM antibody.

11. The method of claim 10, further comprising recovering the anti-HVEM antibody from a host cell culture.

12. A pharmaceutical composition for treating cancer, comprising the antibody of any one of claims 1 to 7 as an active ingredient and a pharmaceutically acceptable carrier.

13. The pharmaceutical composition of claim 12, wherein the cancer is solid tumor or hematologic malignancy.

14. The pharmaceutical composition of claim 12 or 13, wherein the pharmaceutical composition is used in combination with another therapeutic agent.

15. The pharmaceutical composition of claim 14, wherein the other therapeutic agent is an immunotherapeutic agent, a chemotherapeutic agent, or both.

16. The pharmaceutical composition of claim 15, wherein the immunotherapeutic agent is an immune checkpoint inhibitor.

17. The pharmaceutical composition of claim 16, wherein the immune checkpoint inhibitor is a PD-1 antagonist, a PD-L1 antagonist, a CTLA-4 antagonist, or a combination thereof.

18. The pharmaceutical composition of claim 17, wherein the immune checkpoint inhibitor is at least one selected from anti-PD-1 antibodies, anti-PD-L1 antibodies, anti-CTLA4 antibodies, or antigen-binding fragments thereof.

19. The pharmaceutical composition of any one of claims 14 to 18, wherein the pharmaceutical composition is administered as a single or separate formulation simultaneously or sequentially along with another therapeutic agent.

20. A method of treating cancer in a subject, the method comprising administering a therapeutically effective amount of the antibody according to any one of claims 1 to 7 to a subject in need thereof.
